# EUROPEAN PATENT SPECIFICATION

(11) **EP 3 797 820 B1**
(45) Date of publication and mention of the grant of the patent: **03.07.2024**
(21) Application number: 19807065.8
(22) Date of filing: 22.05.2019
(51) Int. Cl.: A61M 37/00

(54) **JAPANESE-ENCEPHALITIS-VACCINE-CONTAINING MICRONEEDLE ARRAY**
JAPAN-ENZEPHALITISIMPFSTOFFHALTIGE MIKRONADELANORDNUNG
RÉSEAU DE MICRO-AIGUILLES CONTENANT UN VACCIN CONTRE L'ENCÉPHALITE JAPONAISE

(30) Priority: 23.05.2018 JP 2018098810
(43) Date of publication of application: 31.03.2021
(73) Proprietor: FUJIFILM Corporation, Tokyo 106-8620 (JP)
(72) Inventor: SHIMADA Toshio, Ashigarakami-gun, Kanagawa 258-8577 (JP); MORI Hisahiro, Ashigarakami-gun, Kanagawa 258-8577 (JP); KUSANO Takayuki, Ashigarakami-gun, Kanagawa 258-8577 (JP)
(74) Representative: Hoffmann Eitle
(86) International application number: PCT/JP2019/020284
(87) International publication number: WO 2019/225650

(56) References cited:
- EP-A1- 2 153 863
- EP-A1- 3 085 357
- WO-A1-2010/074239
- WO-A1-2015/073919
- WO-A1-2017/179614
- WO-A1-2017/179615
- WO-A1-2018/047596
- WO-A1-2018/047596
- JP-A- 2017 047 075
- JP-A- 2017 095 427
- KR-A- 20160 112 975
- US-A- 5 374 243
- US-A1- 2013 287 832

## Description

### Technical Field

The present invention relates to a Japanese encephalitis vaccine-containing microneedle array and, in particular, to an autolytic microneedle array containing a Japanese encephalitis vaccine.

### Background Art

As a drug administration method of administering an appropriate amount of drug and achieving sufficient medicinal effects, a method of using a microneedle array comprising drug-containing microneedles (a needle portion) with a high aspect ratio, by which the microneedles penetrate into a stratum corneum barrier layer to inject the drug into the skin without pain, has attracted attention. For example, an autolytic microneedle array comprising a biosoluble substance as a base material has been reported. In such an autolytic microneedle array, a drug is retained in the base material, and the base material is autolyzed when the microneedles are inserted into the skin, so that the drug can be intracutaneously administered.

Patent Document 1 discloses a method for producing an array as a microstructure, comprising (a) a step of dissolving or suspending at least one therapeutic agent in a solvent to form a therapeutic agent solution or suspension, (b) a step of dissolving at least one polymer in a solvent to form a polymer solution, (c) a step of mixing the therapeutic agent solution or suspension and the polymer solution or suspension to form a polymer matrix solution or suspension, (d) a step of dispensing the polymer matrix solution or suspension into a forming die having a microstructure cavity array, (e) a step of filling the microstructure cavities in the forming die with the solution or suspension, (f) a step of removing an excessive amount of polymer matrix solution or suspension on the surface of the forming die, (g) a step of drying the solution or suspension from the below of the forming die at a temperature of approximately 5°C to 50°C, and (h) a step of drying the microstructure under vacuum at a temperature of approximately 5°C to 50°C.

Patent Document 2 discloses a delivery system for the transcutaneous delivery of an epoetin-based agent to a patient, wherein the delivery system comprises: a microspike member having a plurality of microspikes adapted to penetrate into the stratum corneum of the patient; and a biocompatible coating disposed on the microspike member, wherein the coating is formed from a coating preparation comprising at least one type of epoetin-based agent.

Patent Document 3 discloses a needle-shaped body surface applicable preparation having a base agent consisting of a biosoluble substance and a substance of interest retained on the base agent, wherein the preparation is used by being inserted into the body surface, and the substance of interest is absorbed into the body by dissolution of the base agent. The needle-shaped body surface applicable preparation consists of two or more divisions divided by the interfaces in the insertion direction, and the substance of interest is retained in at least one division other than the endmost division. The division retaining the substance of interest is obtained by dissolving the substance constituting the base agent in a solvent, further dissolving or dispersing the substance of interest in the solvent, and then drying and solidifying the liquid-state base agent containing the substance of interest. The division retaining the substance of interest and the division not retaining the substance of interest each consist of a different composition that has been prepared, separately.

### Prior Art Documents

### Patent Documents

Patent Document 1: Japanese Patent Publication (Kohyo) No. 2016-512754 A
Patent Document 2: Japanese Patent Publication (Kohyo) No. 2008-530230 A
Patent Document 3: Japanese Patent No. 5538897

Furthermore Patent Document WO 2017/179614 A1 discloses a microneedle array.

### Summary of Invention

### Object to be Solved by the Invention

As a result of the studies conducted by the present inventors, it has been found that, among various vaccines, in particular, a Japanese encephalitis vaccine easily loses its activity in a drying step during the production of a microneedle array. In the above-described Patent Documents 1 to 3, studies regarding suppression of a reduction in such activity have not been conducted.

It is an object of the present invention to provide a Japanese encephalitis vaccine-containing microneedle array capable of suppressing a reduction in the activity of a Japanese encephalitis vaccine.

### Means for Solving the Object

The present inventors have conducted intensive studies directed toward achieving the aforementioned object. As a result, the present inventors have found that a reduction in the activity of a Japanese encephalitis vaccine can be suppressed by using a microneedle array having a sheet portion and a plurality of needle portions present on the upper surface of the sheet portion, wherein the needle portion comprises at least one of an electrically neutral water-soluble polymer and a disaccharide, a Japanese encephalitis vaccine, and an electrically neutral surfactant, and the sheet portion comprises at least one of an electrically neutral water-soluble polymer and a disaccharide.

Specifically, the present invention provides the following
(1) A microneedle array having a sheet portion and a plurality of needle portions present on the upper surface of the sheet portion, wherein
   the needle portion comprises at least one of an electrically neutral water-soluble polymer and a disaccharide, a Japanese encephalitis vaccine, and an electrically neutral surfactant, and
   the sheet portion comprises at least one of an electrically neutral water-soluble polymer and a disaccharide.

Preferred embodiments further provide:
(2) The microneedle array according to (1), wherein the electrically neutral surfactant is sorbitan fatty acid ester, a polyoxyethylene/polyoxypropylene copolymer, polyoxyethylene hydrogenated castor oil, or octylphenol ethoxylate.
(3) The microneedle array according to (1) or (2), wherein the weight average molecular weight of the electrically neutral water-soluble polymer comprised in the needle portion is 5,000 or more and 100,000 or less.
(4) The microneedle array according to any one of (1) to (3), wherein the water-soluble polymer comprised in the needle portion is a polysaccharide.
(5) The microneedle array according to any one of (1) to (4), wherein the water-soluble polymer comprised in the needle portion is hydroxyethyl starch.
(6) The microneedle array according to any one of (1) to (5), wherein the weight average molecular weight of the electrically neutral water-soluble polymer comprised in the sheet portion is 5,000 or more and 100,000 or less.
(7) The microneedle array according to any one of (1) to (6), wherein the water-soluble polymer comprised in the sheet portion is a polysaccharide.
(8) The microneedle array according to any one of (1) to (7), wherein the water-soluble polymer comprised in the sheet portion is hydroxyethyl starch.
(9) The microneedle array according to any one of (1) to (8), wherein the disaccharide comprised in the needle portion is sucrose.

### Advantageous Effects of Invention

According to the present invention, a reduction in the activity of a Japanese encephalitis vaccine can be suppressed upon the production of a microneedle array.

### Brief Description of Drawings

Fig. 1A is a perspective view of a conical microneedle, Fig. 1B is a perspective view of a pyramidal microneedle, and Fig. 1C is a cross-sectional view of conical and pyramidal microneedles.
Fig. 2 is a perspective view of a microneedle having another shape.
Fig. 3 is a perspective view of a microneedle having another shape.
Fig. 4 is a cross-sectional view of the microneedles shown in Fig. 2 and Fig. 3.
Fig. 5 is a perspective view of a microneedle having another shape.
Fig. 6 is a perspective view of a microneedle having another shape.
Fig. 7 is a cross-sectional view of the microneedles shown in Fig. 5 and Fig. 6.
Fig. 8 is a cross-sectional view of a microneedle having another shape, in which the inclination (angle) of the lateral surface of the needle portion is continuously changed.
Figs. 9A to 9C show a process chart of a method of producing a mold.
Fig. 10 is an enlarged view of a mold.
Fig. 11 is a cross-sectional view showing a mold having another shape.
Figs. 12A to 12C show a schematic view showing a step of filling a solution containing a Japanese encephalitis vaccine into a mold.
Fig. 13 is a perspective view showing a tip of a nozzle.
Fig. 14 is a partially enlarged view of a nozzle tip and a mold during filling.
Fig. 15 is a partially enlarged view of a nozzle tip and a mold during moving.
Figs. 16A to 16D show an explanatory view of a step of forming another microneedle array.
Figs. 17A to 17C show an explanatory view of a step of forming another microneedle array.
Fig. 18 is an explanatory view showing a peeling step.
Fig. 19 is an explanatory view showing another peeling step.
Fig. 20 is an explanatory view showing a microneedle array.
Figs. 21(A) and 21(B) are a plan view and a side view of an original form, respectively.
Fig. 22 is a schematic diagram of a filling device used in the Examples.

### Embodiment of Carrying out the Invention

Hereinafter, the embodiments of the present invention will be described in detail.

In the present description, the phrase "comprise a Japanese encephalitis vaccine" means that a needle comprises a Japanese encephalitis vaccine in an amount sufficient for exhibiting the effects of the Japanese encephalitis vaccine, when the needle is punctured into the body surface.

### [Configuration of microneedle array]

The microneedle array of the present invention is a microneedle array having a sheet portion and a plurality of needle portions present on the upper surface of the sheet portion, wherein the needle portion comprises at least one of an electrically neutral water-soluble polymer and a disaccharide, a Japanese encephalitis vaccine, and an electrically neutral surfactant, and the sheet portion comprises at least one of an electrically neutral water-soluble polymer and a disaccharide.

In the present invention, the term "a plurality of" means one or more.

In order to efficiently administer a Japanese encephalitis vaccine into the skin, the microneedle array of the present invention comprises, at least, a sheet portion and a needle portion, and the Japanese encephalitis vaccine is deposited on the needle portion.

The microneedle array of the present invention is a device in which a plurality of needle portions are arranged in an array on the upper surface side of a sheet portion. The needle portions are preferably arranged on the upper surface side of the sheet portion. The needle portions may be directly arranged on the upper surface of the sheet portion, or the needle portions may also be arranged on the upper surface of a frustum portion disposed on the upper surface of the sheet portion.

Preferably, the needle portions are arranged on the upper surface of the frustum portion disposed on the upper surface of the sheet portion. In this case, the microneedle array of the present invention has a plurality of frustum portions between a sheet portion and a plurality of needle portions. In this aspect, it is preferable that the tip region of each needle portion comprises at least one of an electrically neutral water-soluble polymer and a disaccharide, Japanese encephalitis vaccine, and an electrically neutral surfactant, and that the root region of the needle portion other than the tip region, the frustum portion, and the sheet portion comprise at least one of an electrically neutral water-soluble polymer and a disaccharide. The water-soluble polymers comprised in the needle portion and the sheet portion may be identical to or different from each other. The water-soluble polymer will be described later.

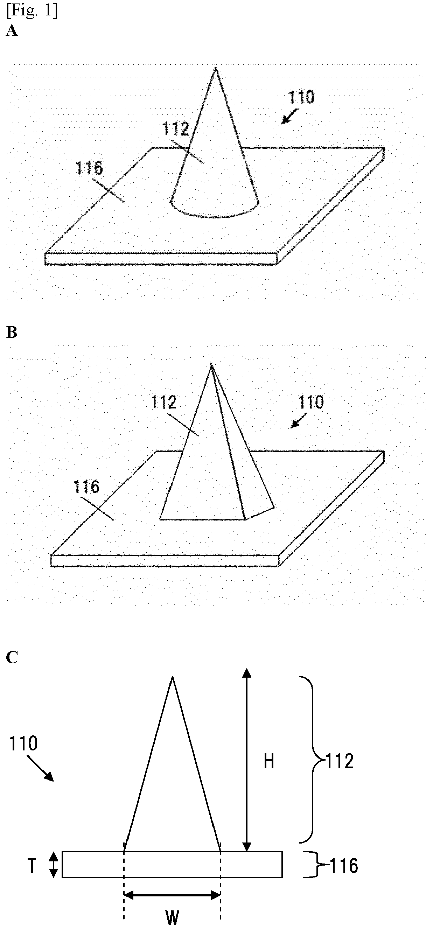

The sheet portion is a base for supporting the needle portions, and has a plane shape like a sheet portion 116 as shown in Fig. 1 to Fig. 8. In this context, the upper surface of the sheet portion indicates a surface on which a plurality of needle portions are arranged in an array.

The area of the sheet portion is not particularly limited, and it is preferably 0.005 to 1000 mm², more preferably 0.05 to 500 mm², and further preferably 0.1 to 400 mm².

The thickness of the sheet portion is indicated as a distance between the surface contacted with the frustum portion or the needle portion and the surface of the opposite side. The thickness of the sheet portion is preferably 1 µm or more and 2000 µm or less, more preferably 3 µm or more and 1500 µm or less, and further preferably 5 µm or more and 1000 µm or less.

According to the invention, the sheet portion comprises at least one of an electrically neutral water-soluble polymer and a disaccharide. The sheet portion may also comprise additives other than the aforementioned components (for example, a water-soluble polymer having an electric charge, etc.). It is to be noted that the sheet portion preferably does not comprise a Japanese encephalitis vaccine.

The electrically neutral water-soluble polymer comprised in the sheet portion is not particularly limited. Examples of the electrically neutral water-soluble polymer may include polysaccharide, polyvinylpyrrolidone, polyoxyethylene polyoxypropylene glycol, polyethylene glycol, and polyvinyl alcohol. Examples of the above-described polysaccharide may include Pullulan, dextran, dextrin, cellulose derivatives (e.g., water-soluble cellulose derivatives in which cellulose is partially modified, such as hydroxypropyl cellulose or hydroxypropylmethyl cellulose), hydroxyethyl starch, and gum Arabic. The above-described components may be used alone as a single type, or may also be used as a mixture of two or more types.

Among the above-described components, the electrically neutral water-soluble polymer comprised in the sheet portion is preferably at least one selected from the group consisting of hydroxyethyl starch, dextran, polyvinylpyrrolidone, polyoxyethylene polyoxypropylene glycol, polyethylene glycol and polyvinyl alcohol, and the electrically neutral water-soluble polymer is particularly preferably hydroxyethyl starch.

In the present invention, the sheet portion may also further comprise a non-electrically neutral water-soluble polymer, or sugars other than disaccharides. Examples of the non-electrically neutral water-soluble polymer or the sugars other than disaccharides comprised in the sheet portion may include hyaluronic acid, sodium hyaluronate, chondroitin sulfate, sodium chondroitin sulfate, and carboxymethyl cellulose.

The content percentage of the non-electrically neutral water-soluble polymer or the sugars other than disaccharides in the sheet portion is preferably 0% by mass or more and less than 50% by mass, more preferably 0% by mass or more and less than 40% by mass, further preferably 0% by mass or more and 30% by mass or less, and most preferably 0% by mass or more and 20% by mass or less, based on the total solid content of the sheet portion.

The weight average molecular weight of the electrically neutral water-soluble polymer comprised in the sheet portion is preferably 5,000 or more and 100,000 or less, more preferably 10,000 or more and 100,000 or less, and further preferably 30,000 or more and 90,000 or less.

The sheet portion may comprise a disaccharide. Examples of the disaccharide may include sucrose, lactulose, lactose, maltose, trehalose, and cellobiose. Among these, sucrose is particularly preferable.

The microneedle array is constituted with a plurality of needle portions arranged in an array on the upper surface side of the sheet portion. The needle portion is a convex structure having a tip. The needle is not limited to a needle having a sharp tip, but it may also be a needle having a blunt tip.

Examples of the shape of the needle portion may include a cone, a pyramid (a square pyramid, etc.), and a spindle. The needle portion may have, for example, a shape such as a needle portion 112 shown in Fig. 1 to Fig. 8, and the entire shape of the needle portion may be a cone or a pyramid (a square pyramid, etc.), or may also be a structure in which the inclination (angle) of the needle portion lateral surface is continuously changed. Otherwise, the needle portion may also have a multilayer structure consisting of two or more layers, in which the inclination (angle) of the lateral surface is discontinuously changed.

When the microneedle array of the present invention is applied to the skin, it is preferable that the needle portion is inserted into the skin, so that the upper surface of the sheet portion or a part thereof is allowed to come into contact with the skin.

The height (length) of the needle portion indicates the length of a perpendicular line dropped from the tip of the needle portion to the frustum portion or the sheet portion (in a case where such a frustum portion is absent). The height (length) of the needle portion is not particularly limited, and it is preferably 50 µm or more and 3000 µm or less, more preferably 100 µm or more and 1500 µm or less, and further preferably 100 µm or more and 1000 µm or less. When the length of the needle portion is 50 µm or more, transdermal administration of a Japanese encephalitis vaccine can be carried out. On the other hand, when the length of the needle portion is 3000 µm or less, the development of pain caused by the contact of the needle portion with a nerve can be prevented, and bleeding can be avoided. Thus, the length of the needle portion is preferably within the aforementioned range.

The interface between the frustum portion (if the frustum portion is absent, it is the needle portion,) and the sheet portion is referred to as a "base portion." The distance between the farthest points in the base of a single needle portion is preferably 50 µm or more and 2000 µm or less, more preferably 100 µm or more and 1500 µm or less, and further preferably 200 µm or more and 1000 µm or less.

The number of the needle portions arranged on a single microneedle array is preferably 1 to 2000, more preferably 3 to 1000, and further preferably 5 to 500. When two needle portions are comprised in a single microneedle array, the interval between the two needle portions is indicated by the distance between the legs of perpendicular lines dropped from the tips of the needle portions to the frustum portion or the sheet portion (in a case where such a frustum portion is absent). When three or more needle portions are comprised in a single microneedle array, with regard to the intervals between the arranged needle portions, the distance between the legs of perpendicular lines that are dropped from the tips of the two closest needle portions out of all of the needle portions to the frustum portion or the sheet portion (in a case where such a frustum portion is absent) is obtained, and a mean value of the obtained distances is then obtained. The interval between the needle portions is preferably 0.1 mm or more and 10 mm or less, more preferably 0.2 mm or more and 5 mm or less, and further preferably 0.3 mm or more and 3 mm or less.

According to the invention, the needle portion contains at least one of an electrically neutral water-soluble polymer and a disaccharide, a Japanese encephalitis vaccine, and an electrically neutral surfactant.

In order for the needle portion not to affect a human body even if it remains in the skin, the electrically neutral water-soluble polymer is preferably a biosoluble substance.

The electrically neutral water-soluble polymer comprised in the needle portion is not particularly limited. Examples of the electrically neutral water-soluble polymer may include polysaccharide, polyvinylpyrrolidone, polyoxyethylene polyoxypropylene glycol, polyethylene glycol, and polyvinyl alcohol. Examples of the above-described polysaccharide may include Pullulan, dextran, dextrin, cellulose derivatives (e.g., water-soluble cellulose derivatives in which cellulose is partially modified, such as hydroxypropyl cellulose or hydroxypropylmethyl cellulose), hydroxyethyl starch, and gum Arabic. The above-described components may be used alone as a single type, or may also be used as a mixture of two or more types.

Among the above-described components, the water-soluble polymer comprised in the needle portion is preferably at least one selected from the group consisting of hydroxyethyl starch, dextran, polyvinylpyrrolidone, polyoxyethylene polyoxypropylene glycol, polyethylene glycol, and polyvinyl alcohol, and the water-soluble polymer is particularly preferably hydroxyethyl starch. Furthermore, in order to hinder agglutination of the water-soluble polymer upon the mixing with a Japanese encephalitis vaccine, in general, a polysaccharide having no electric charge is more preferable. The electrically neutral water-soluble polymer comprised in the needle portion may be identical to or different from the electrically neutral water-soluble polymer comprised in the sheet portion.

The weight average molecular weight of the electrically neutral water-soluble polymer comprised in the needle portion is preferably 5,000 or more and 100,000 or less, more preferably 10,000 or more and 100,000 or less, and further preferably 30,000 or more and 90,000 or less.

The needle portion (in particular, the tip region of the needle portion) may comprise a disaccharide. Examples of the disaccharide may include sucrose, lactulose, lactose, maltose, trehalose, and cellobiose. Among these, sucrose is particularly preferable.

In the present invention, the content percentage of the electrically neutral water-soluble polymer or the disaccharide is 50% by mass or more of the total solid content of the needle portion. The content percentage of the electrically neutral water-soluble polymer or the disaccharide is preferably 55% by mass or more, more preferably 60% by mass or more, and further preferably 65% by mass or more, based on the total solid content of the needle portion.

The upper limit of the content percentage of the electrically neutral water-soluble polymer or the disaccharide is not particularly limited. The upper limit of the content percentage of the electrically neutral water-soluble polymer or the disaccharide is preferably 99% by mass or less, more preferably 95% by mass or less, and further preferably 90% by mass or less, based on the total solid content of the needle portion.

By setting the content percentage of the electrically neutral water-soluble polymer or the disaccharide to be 50% by mass or more based on the total solid content of the needle portion, favorable puncture ability and favorable effects can be obtained.

The content percentage of the electrically neutral water-soluble polymer or the disaccharide in the total solid content of the needle portion can be measured by the following method, but the measurement method is not particularly limited thereto. As a measurement method, for example, the needle portion is cut from the produced microneedle array, the needle portion is then dissolved in a buffer solution (a buffer solution suitable for dissolving the water-soluble polymer constituting the needle portion, such as a phosphate buffered saline (PBS)), and the amount of the electrically neutral water-soluble polymer or the disaccharide in the solution can be measured according to a high performance liquid chromatographic method.

According to the invention, the needle portion contains a Japanese encephalitis vaccine.

As such a Japanese encephalitis vaccine, a vaccine comprising, as an active ingredient, an inactivated Japanese encephalitis virus can be used. For example, a formalin inactivated virus preparation prepared from a culture solution of Vero cells (African green monkey kidney-derived cell line) infected with a Japanese encephalitis virus, or a formalin inactivated virus preparation prepared from the mouse brain infected with a Japanese encephalitis virus, or the like can be used.

The content of the Japanese encephalitis vaccine in the needle portion is not particularly limited. It is preferably 0.001% to 10% by mass, more preferably 0.003% to 1% by mass, and particularly preferably 0.005% to 0.5% by mass, with respect to the solid content mass of the needle portion.

The content of the Japanese encephalitis vaccine in a single microneedle array is not particularly limited. It is preferably 0.05 to 5 µg, and more preferably 0.2 to 3 µg, at a protein content.

According to the invention, the needle portion contains an electrically neutral surfactant (nonionic surfactant).

Examples of the electrically neutral surfactant may include sugar alcohol fatty acid esters such as sucrose fatty acid ester, sorbitan fatty acid ester, glycerin fatty acid ester, polyglycerin fatty acid ester, propylene glycol fatty acid ester, polyoxyethylene sorbitan fatty acid ester, polyoxyethylene glycerin fatty acid ester, polyethylene glycol fatty acid ester, a polyoxyethylene/polyoxypropylene copolymer, polyoxyethylene castor oil, polyoxyethylene hydrogenated castor oil, and octylphenol ethoxylate. Among the above-described substances, sorbitan fatty acid ester, a polyoxyethylene/polyoxypropylene copolymer, or polyoxyethylene hydrogenated castor oil are particularly preferable. As such electrically neutral surfactants, commercially available products such as Tween (registered trademark) 80, MONTANOX (registered trademark) 80 API, Pluronic (registered trademark) F-68, NIKKOL (registered trademark) HCO-60, or Triton (registered trademark) X-100 can also be used.

The additive amount of the electrically neutral surfactant is not particularly limited, and it is preferably 0.01 µg or more, and more preferably 0.1 µg or more, for a single microneedle array (area: 1 cm²).

Hereinafter, preferred embodiments of the present invention will be described with reference to the drawings attached herewith. However, the present invention is not limited thereto.

Fig. 1 to Fig. 8 each show a microneedle 110 that is a partially enlarged view of a microneedle array. The microneedle array of the present invention is configured by forming a plurality of needle portions 112 on the surface of a sheet portion 116. (In the figures, only one needle portion 112, or one frustum portion 113 and one needle portion 112 are shown on the sheet portion 116, and these are referred to as a microneedle 110).

In Fig. 1A, the needle portion 112 has a conical shape, whereas in Fig. 1B, the needle portion 112 has a square pyramidal shape. In Fig. 1C, H indicates the height of the needle portion 112, W indicates the diameter (width) of the needle portion 112, and T indicates the height (thickness) of the sheet portion 116.

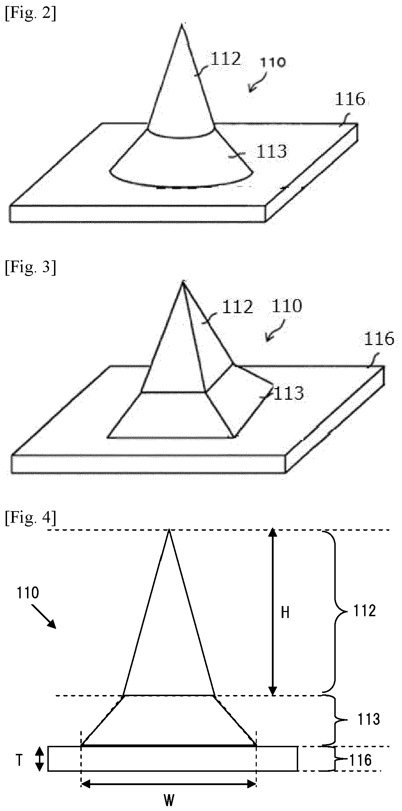

Fig. 2 and Fig. 3 each show a microneedle 110 having another shape, in which a frustum portion 113 and a needle portion 112 are formed on the surface of a sheet portion 116. In Fig. 2, the frustum portion 113 has a truncated conical shape, and the needle portion 112 has a conical shape. On the other hand, in Fig. 3, the frustum portion 113 has a truncated square pyramidal shape, and the needle portion 112 has a square pyramidal shape. However, the shape of the needle portion is not limited to these shapes.

Fig. 4 is a cross-sectional view of the microneedles shown in Fig. 2 and Fig. 3. In Fig. 4, H indicates the height of a needle portion 112, W indicates the diameter (width) of a base portion, and T indicates the height (thickness) of a sheet portion 116.

The microneedle array of the present invention preferably takes the shape of the microneedle 110 of Fig. 4, rather than the shape of the microneedle 110 of Fig. 1C. By taking such a structure, the entire volume of the needle portion is increased, and upon the production of the microneedle array, more Japanese encephalitis vaccines can be concentrated into the upper tip of the needle portion.

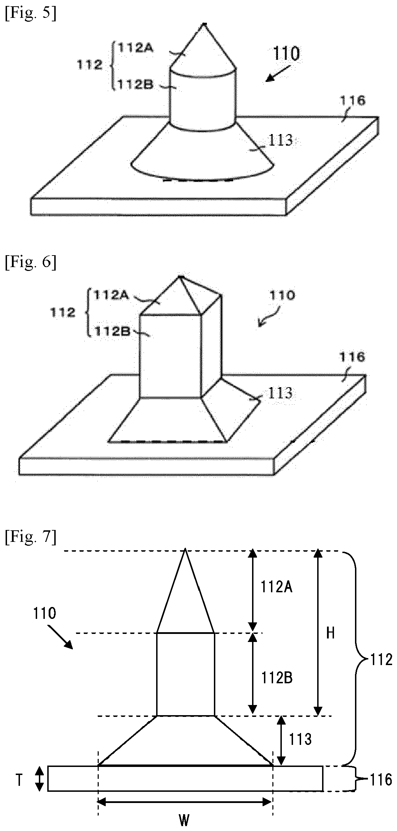

Fig. 5 and Fig. 6 each show a microneedle 110 having still another shape.

A needle portion first layer 112A shown in Fig. 5 has a conical shape, and a needle portion second layer 112B has a columnar shape. A needle portion first layer 112A shown in Fig. 6 has a square pyramidal shape, and a needle portion second layer 112B shown in Fig. 6 has a square columnar shape. However, the shape of the needle portion is not limited to these shapes.

Fig. 7 is a cross-sectional view of the microneedles shown in Fig. 5 and Fig. 6. In Fig. 7, H indicates the height of a needle portion 112, W indicates the diameter (width) of a base portion, and T indicates the height (thickness) of a sheet portion 116.

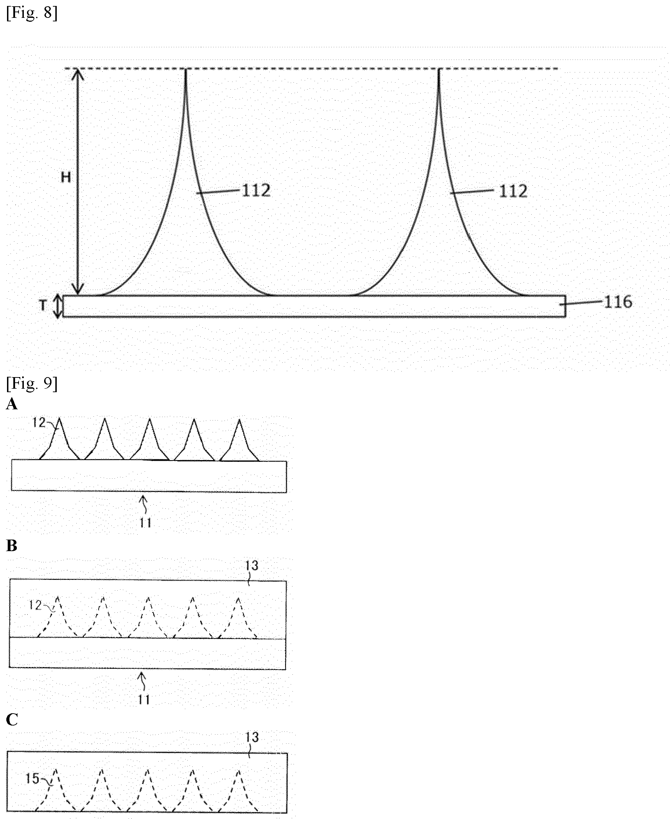

Fig. 8 is a cross-sectional view of a microneedle having another shape, in which the inclination (angle) of the lateral surface of a needle portion 112 is continuously changed. In Fig. 8, H indicates the height of a needle portion 112, and T indicates the height (thickness) of a sheet portion 116.

In the microneedle array of the present invention, with regard to the lateral line of the needle portion, microneedles are preferably arranged with intervals of approximately 0.1 to 10 needles per mm. The microneedle array more preferably has 1 to 10000 microneedles per cm². The microneedles can be efficiently punctured into the skin by setting the density of the microneedles at 1 microneedle/cm² or more. On the other hand, by setting the density of the microneedles to be 10000 microneedles/cm² or less, it becomes possible for the microneedles to be sufficiently punctured into the skin. The density of the needle portion is preferably 10 to 5000 microneedles/cm², more preferably 25 to 1000 microneedles/cm², and particularly preferably 25 to 400 microneedles/cm².

The microneedle array of the present invention can be supplied in the form in which it is hermetically preserved together with a desiccant. As such desiccants, known desiccants (e.g., silica gel, quicklime, calcium chloride, silica alumina, a sheet-shaped desiccant, etc.) can be used.

### [Method for producing microneedle array]

The microneedle array of the present invention can be produced by the following method, for example, in accordance with the method described in Japanese Patent Publication (Kokai) No. 2013-153866 A or International Publication No. WO 2014/077242.

### (Production of mold)

Figs. 9A to 9C show a process chart of producing a mold (die). As shown in Fig. 9A, an original form for producing a mold is first produced. There are two type of methods for producing an original form 11.

According to the first method, photoresist is applied onto a Si substrate, followed by exposure and development. Thereafter, etching is carried out by RIE (reactive ion etching), etc., so that an array of conical shaped portions (protrusions) 12 is produced on the surface of an original form 11. Besides, when etching such as RIE is carried out to form conical shaped portions on the surface of the original form 11, the etching is carried out from an oblique direction, while rotating the Si substrate, so that the conical shape can be formed. The second method is a method of forming an array of shaped portions 12 such as square pyramids on the surface of an original form 11 by processing a metal substrate such as Ni using a cutting tool such as a diamond bit.

Subsequently, a mold is produced. Specifically, as shown in Fig. 9B, a mold 13 is produced from the original form 11. There are the following 4 methods.

The first method is a method comprising pouring a silicone resin prepared by adding a hardening agent to PDMS (polydimethylsiloxane; for example, SYLGARD 184 (registered trademark) manufactured by Dow Corning) into the original form 11, then subjecting the original form to a heat treatment at 100°C to harden it, and then peeling the hardened resin from the original form 11. The second method is a method comprising pouring a UV (ultraviolet) curable resin hardened by ultraviolet irradiation into the original form 11, then applying ultraviolet ray to the original form in a nitrogen atmosphere, and then peeling the resin from the original form 11. The third method is a method comprising pouring a solution prepared by dissolving a plastic resin such as polystyrene or PMMA (polymethyl methacrylate) in an organic solvent into an original form 11 coated with a peeling agent, then drying the original form to volatilize the organic solvent and harden the residue, and then peeling the hardened resin from the original form 11. The fourth method is a method of producing an inverted product by Ni electroforming.

Thereby, produced is a mold 13, in which needle recess portions 15 that are inverted shapes of the conical or pyramidal shapes of the original form 11 are arranged in a two-dimensional array. The thus produced mold 13 is shown in Fig. 9C.

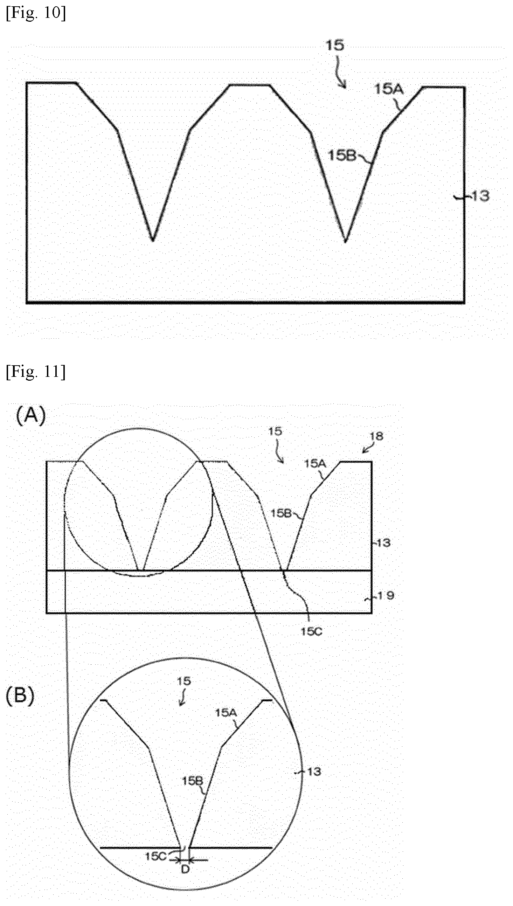

Fig. 10 shows another preferred aspect of the mold 13. The needle recess portion 15 comprises a tapered inlet 15A that narrows from the surface of the mold 13 into the depth direction, and a tip recess portion 15B that tapers in the depth direction. By allowing the inlet 15A to have a tapered shape, a solution is easily filled into the needle recess portion 15.

Fig. 11 shows an aspect of a mold composite 18 that is more preferable for the production of a microneedle array. In Fig. 11, part (A) shows a mold composite 18. In Fig. 11, part (B) shows an enlarged view of the part enclosed with a circle in the part (A).

As shown in Fig. 11(A), the mold composite 18 comprises a mold 13 in which an air vent hole 15C is formed at the tip (bottom) of a needle recess portion 15, and a gas permeation sheet 19 that is adhered to the back surface of the mold 13 and is formed with a material through which gas permeates but liquid does not permeate. The air vent hole 15C is formed as a through hole penetrating into the back surface of the mold 13. Herein, the back surface of the mold 13 means the surface on the side on which the air vent hole 15C is formed. Thereby, the tip of the needle recess portion 15 is communicated with the atmosphere via the air vent hole 15C and the gas permeation sheet 19.

By using such a mold composite 18, a solution filling into the needle recess portion 15 does not permeate, and only the air existing in the needle recess portion 15 can be discharged from the needle recess portion 15. Thereby, the transcription ability of transcribing the shape of the needle recess portion 15 into a polymer can be improved, so that a sharper needle portion can be formed.

The diameter D of the air vent hole 15C is preferably in the range of 1 to 50 µm. When the diameter D of the air vent hole 15C is less than 1 µm, it cannot sufficiently play a role as an air vent hole. On the other hand, when the diameter D of the air vent hole 15C exceeds 50 µm, the sharpness of the tip of the molded microneedle is impaired.

As a gas permeation sheet 19 formed with a material through which gas permeates but liquid does not permeate, for example, a gas permeable film (manufactured by Sumitomo Electric Industries, Ltd., Poreflon (registered trademark), FP-010) can be preferably used.

As a material used for the mold 13, an elastic material or a metallic material can be used, and an elastic material is preferable. A material having high gas permeability is more preferable. Oxygen permeability that is representative gas permeability is preferably 1 × 10⁻¹² (mL/s • m²·Pa) or more, and is more preferably 1 × 10⁻¹⁰ (mL/s·m² • Pa) or more. Besides, 1 mL is 10⁻⁶ m³. By setting gas permeability within the above-described range, the air existing in the recess portion of the mold 13 can be discharged from the mold side, so that a microneedle array with few defects can be produced. Specific examples of such a material may include materials, such as silicone resins (e.g., SYLGARD 184 (registered trademark) manufactured by Dow Corning, and KE-1310ST (product number) manufactured by Shin-Etsu Chemical Co., Ltd.), UV curable resins, and plastic resins (e.g., polystyrene and PMMA (polymethyl methacrylate)), which are melted or dissolved in a solvent. Among these materials, silicone rubber-based materials are preferable because they have durability to transcription by repeated pressurization, and also have good peelability from the materials. Moreover, examples of the metallic material may include Ni, Cu, Cr, Mo, W, Ir, Tr, Fe, Co, MgO, Ti, Zr, Hf, V, Nb, Ta, α-aluminum oxide, zirconium oxide, stainless steel (e.g., STAVAX (trademark) material manufactured by Bohler-Uddeholm KK), and an alloy thereof. As a material for a frame 14, the same material as that for the mold 13 can be used.

### (Solution)

It is preferable to prepare:
(i) a solution comprising at least one of an electrically neutral water-soluble polymer and a disaccharide, a Japanese encephalitis vaccine, and an electrically neutral surfactant, which is for use in forming a needle portion tip region that is a part of a needle portion, and
(ii) a solution comprising at least one of an electrically neutral water-soluble polymer and a disaccharide, which is for use in forming a needle portion root region other than the needle portion tip region in the needle portion, and a sheet portion (or a needle portion root region other than the needle portion tip region in the needle portion, a frustum portion, and a sheet portion).

The types of the electrically neutral water-soluble polymer, the disaccharide, and the electrically neutral surfactant are as described above in the present description.

The concentration of the electrically neutral water-soluble polymer or the disaccharide in the above-described solution is different depending on the types of substances used. In general, it is preferably 1% to 50% by mass. In addition, the solvent used in dissolution may also be a solvent having volatility, even other than water, and methyl ethyl ketone (MEK), alcohol or the like can be used.

### (Formation of needle portion tip region)

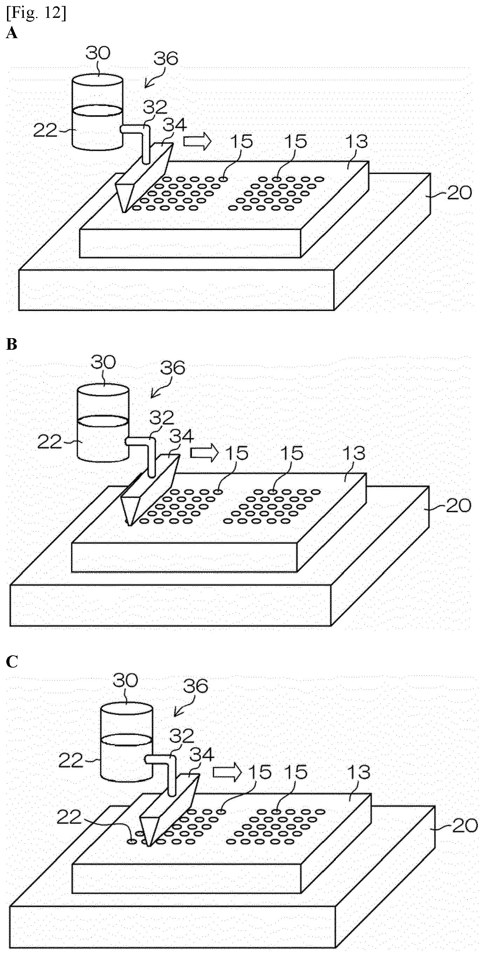

As shown in Fig. 12A, a mold 13 having two-dimensionally arrayed needle recess portions 15 is disposed on a base 20. On the mold 13, a couple of two-dimensionally arrayed, multiple (5 x 5) needle recess portions 15 are formed. Here is prepared a liquid supplying device 36 having a tank 30 for storing a solution 22 comprising a Japanese encephalitis vaccine, a pipe 32 connected with the tank, and a nozzle 34 connected with the tip of the pipe 32. In the present example, a case where 5 x 5 needle recess portions 15 are two-dimensionally arrayed is exemplified. However, the number of the needle recess portions 15 is not limited to 5 x 5, and the needle recess portions 15 may be two-dimensionally arrayed with a number of M x N (wherein M and N each independently represent any given integer of 1 or greater, and the integer is preferably 2 to 30, more preferably 3 to 25, and further preferably 3 to 20).

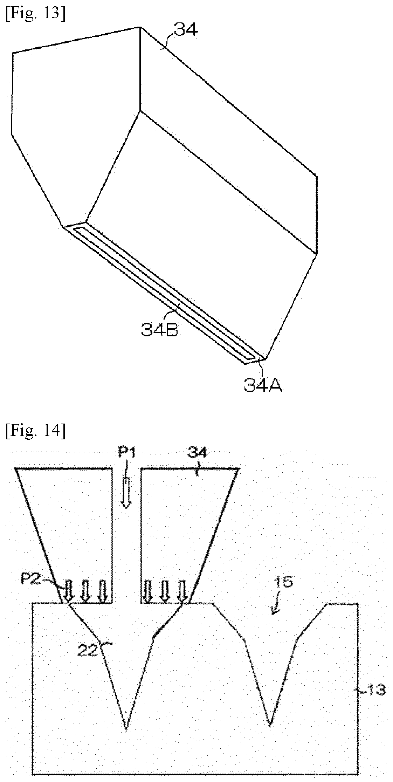

Fig. 13 shows a schematically perspective view of a nozzle tip portion. As shown in Fig. 13, the nozzle 34 comprises, at the tip thereof, a lip portion 34A that is a flat surface and a slit-shaped opening 34B. Using the slit-shaped opening 34B, it becomes possible to simultaneously fill the solution 22 comprising a Japanese encephalitis vaccine into, for example, a plurality of needle recess portions 15 that constitute a line. The size (length and width) of the opening 34B is selected, as appropriate, depending on the number of needle recess portions 15 to be filled with the solution all at once. By increasing the length of the opening 34B, more needle recess portions 15 can be filled with the solution 22 comprising a Japanese encephalitis vaccine all at once. Thereby, it becomes possible to improve productivity.

As a material used for the nozzle 34, an elastic material or a metallic material can be used. Examples of the material may include Teflon (registered trademark), stainless steel (SUS (Steel Use Stainless)), and titanium.

As shown in Fig. 12B, the position of the opening 34B of the nozzle 34 is adjusted on the needle recess portions 15. The lip portion 34A of the nozzle 34 is contacted with the surface of the mold 13. From the liquid supplying device 36, the solution 22 comprising a Japanese encephalitis vaccine is supplied to the mold 13, and the solution 22 comprising a Japanese encephalitis vaccine is filled from the opening 34B of the nozzle 34 into the needle recess portions 15. In the present embodiment, the solution 22 comprising a Japanese encephalitis vaccine is simultaneously filled into a plurality of needle recess portions 15 constituting a line. However, the embodiment is not limited thereto, and it can be configured to fill the solution, one by one, into the needle recess portions 15.

When the mold 13 is formed with a material having gas permeability, the solution 22 comprising a Japanese encephalitis vaccine can be suctioned by suctioning from the back surface of the mold 13, so that the filling of the solution 22 comprising a Japanese encephalitis vaccine into the needle recess portions 15 can be promoted.

After the filling step has been carried out with reference to Fig. 12B, as shown in Fig. 12C, while the lip portion 34A of the nozzle 34 is contacted with the surface of the mold 13, the liquid supplying device 36 is relatively moved in a direction vertical to the length direction of the opening 34B, so that the nozzle 34 is moved to needle recess portions 15, into which the solution 22 comprising a Japanese encephalitis vaccine has not yet been filled. The position of the opening 34B of the nozzle 34 is adjusted onto the needle recess portions 15. In the present embodiment, the example of moving the nozzle 34 is explained, but it may also be possible to move the mold 13.

Since the liquid supplying device is moved while the lip portion 34A of the nozzle 34 is contacted with the surface of the mold 13, the nozzle 34 can scrape the solution 22 comprising a Japanese encephalitis vaccine remaining on the surface of the mold 13 other than the needle recess portions 15. Thus, the solution 22 comprising a Japanese encephalitis vaccine cannot remain on the surface of the mold 13 other than the needle recess portions 15.

In order to reduce damage to the mold 13 and also in order to suppress the deformation of the mold 13 caused by compression as much as possible, the pressure of pressing the nozzle 34 to the mold 13 is preferably as small as possible, upon the moving of the liquid supplying device. In addition, in order to prevent the solution 22 comprising a Japanese encephalitis vaccine from remaining on the mold 13 other than the needle recess portions 15, at least one of the mold 13 or the nozzle 34 is desirably formed with a material that flexibly elastically deforms.

By repeating the filling step shown in Fig. 12B and the moving step shown in Fig. 12C, the solution 22 comprising a Japanese encephalitis vaccine is filled into the 5 x 5 two-dimensionally arrayed needle recess portions 15. After the 5 x 5 two-dimensionally arrayed needle recess portions 15 have been filled with the solution 22 comprising a Japanese encephalitis vaccine, the liquid supplying device 36 is moved to the adjacent 5 x 5 two-dimensionally arrayed needle recess portions 15, and then, the filling step shown in Fig. 12B and the moving step shown in Fig. 12C are repeated. Thus, the solution 22 comprising a Japanese encephalitis vaccine is also filled into the adjacent 5 x 5 two-dimensionally arrayed needle recess portions 15.

The aforementioned filling step and moving step may have an aspect (1) in which the solution 22 comprising a Japanese encephalitis vaccine is filled into the needle recess portions 15, while moving the nozzle 34, or may also have an aspect (2) in which the nozzle 34 is once allowed to stand still on the needle recess portions 15 during the moving of the nozzle 34, the solution 22 comprising a Japanese encephalitis vaccine is then filled into the needle recess portions 15, and after the filling of the solution, the nozzle is moved again. Between the filling step and the moving step, the lip portion 34A of the nozzle 34 is contacted with the surface of the mold 13.

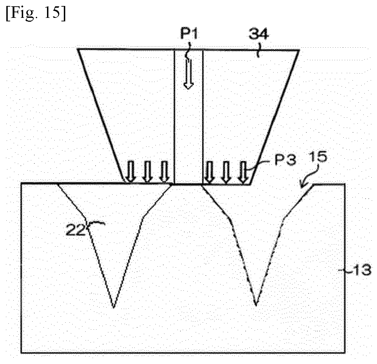

Fig. 14 is a partially enlarged view of the tip of a nozzle 34 and a mold 13 during the filling of a solution 22 comprising a Japanese encephalitis vaccine into needle recess portions 15. As shown in Fig. 15, by adding a pressurization power P1 into a nozzle 34, the filling of a solution 22 comprising a Japanese encephalitis vaccine into needle recess portions 15 can be promoted. Furthermore, when the solution 22 comprising a Japanese encephalitis vaccine is filled into needle recess portions 15, a pressing force P2 necessary for contacting the nozzle 34 with the surface of the mold 13 is preferably set to be the pressurization power P1 in the nozzle 34 or greater. By applying the pressing force P2 ≥ the pressurization power P1, the leakage of the solution 22 comprising a Japanese encephalitis vaccine from the needle recess portions 15 onto the surface of the mold 13 can be suppressed.

Fig. 15 is a partially enlarged view of the tip of the nozzle 34 and the mold 13 during the moving of the nozzle 34. When the nozzle 34 is moved relatively to the mold 13, the pressing force P3 necessary for contacting the nozzle 34 with the surface of the mold 13 is preferably set to be smaller than the pressing force P2 for contacting the nozzle 34 with the surface of the mold 13 during the filling. This is because damage to the mold 13 is reduced and the deformation of the mold 13 due to compression is suppressed.

After the filling of the solution into the 5 x 5 multiple needle recess portions 15 has been completed, the nozzle 34 is moved to the adjacent 5 x 5 multiple needle recess portions 15. Regarding solution supply, when the liquid supplying device is moved to the adjacent 5 x 5 multiple needle recess portions 15, it is preferable to terminate the supply of the solution 22 comprising a Japanese encephalitis vaccine. There is a certain distance from the needle recess portion 15 on the 5th line to the next first needle recess portions 15. When the nozzle 34 is moved between them, if the solution 22 comprising a Japanese encephalitis vaccine is continuously supplied, the liquid pressure in the nozzle 34 becomes excessively high in some cases. As a result, there may be a case where the solution 22 comprising a Japanese encephalitis vaccine is flown out of the nozzle 34 to the portion of the mold 13 other than the needle recess portions 15. In order to suppress this problem, the liquid pressure in the nozzle 34 is detected and if the nozzle is determined to have an excessively high liquid pressure, it is preferable to terminate the supply of the solution 22 comprising a Japanese encephalitis vaccine.

Besides, in the above description, a method of supplying a solution comprising a Japanese encephalitis vaccine by using a dispenser having a nozzle is explained, but bar coating, spin coating, coating using a spray, etc. can also be applied, as well as the coating using a dispenser.

It is preferable to perform a drying treatment, after a solution comprising a Japanese encephalitis vaccine has been supplied to needle recess portions.

Preferably, the microneedle array of the present invention can be produced by: a step of drying a mold for forming needle portions filled with a solution comprising a Japanese encephalitis vaccine to form parts of the needle portions; and a step of filling a solution comprising at least one of an electrically neutral water-soluble polymer and a disaccharide into the upper surfaces of the above-formed parts of the needle portions, followed by drying.

The conditions for drying the mold for forming needle portions filled with a solution comprising a Japanese encephalitis vaccine are preferably conditions in which the water content percentage of the above-described solution reaches 20% or less at 30 minutes to 300 minutes after initiation of the drying.

Particularly preferably, the above-described drying can be regulated, so that the temperature is kept to a temperature at which the activity of the Japanese encephalitis vaccine is not lost, or lower, and the water content percentage of the solution reaches 20% or less at 60 minutes after initiation of the drying.

A method of regulating the speed of the above-described drying may be, for example, any given means capable of delaying the drying, such as temperature, humidity, drying air volume, the use of a container, the volume and/or shape of the container, etc.

The drying can be carried out in a state in which the mold for forming needle portions filled with a solution comprising a Japanese encephalitis vaccine is covered with a container or is stored in a container.

The temperature applied upon the drying is preferably 1°C to 45°C, and more preferably 1°C to 40°C.

The relative humidity applied upon the drying is preferably 10% to 95%, more preferably 20% to 95%, and further preferably 30% to 95%.

### (Formation of sheet portion)

Regarding a step of forming a sheet portion, several aspects will be described.

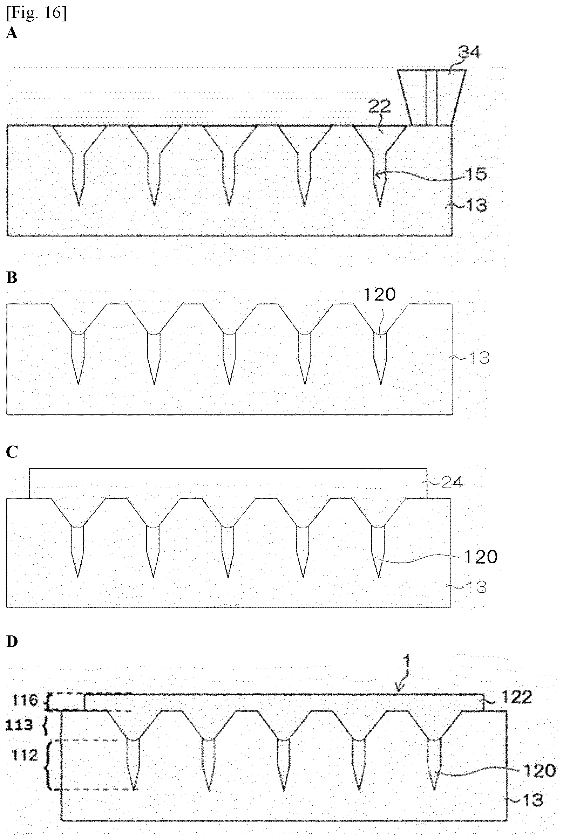

Regarding the step of forming a sheet portion, a first aspect is described with reference to Fig. 16A to Fig. 16D. Into needle recess portions 15 of a mold 13, a solution 22 comprising a Japanese encephalitis vaccine is filled through a nozzle 34. Subsequently, as shown in Fig. 16B, the solution 22 comprising a Japanese encephalitis vaccine is dried and solidified, so that layers 120 comprising the Japanese encephalitis vaccine are formed in the needle recess portions 15. Subsequently, as shown in Fig. 16C, using a dispenser, a solution 24 comprising at least one of an electrically neutral water-soluble polymer and a disaccharide is applied onto the mold 13 on which the layers 120 comprising the Japanese encephalitis vaccine are formed. In addition to the coating using a dispenser, bar coating, spin coating, coating using a spray, etc. can be applied. Since the layers 120 comprising the Japanese encephalitis vaccine are solidified, diffusion of the Japanese encephalitis vaccine in the solution 24 can be suppressed. Subsequently, as shown in Fig. 17D, the solution 24 is dried and solidified, so as to form a microneedle array 1 composed of a plurality of needle portions 112, frustum portions 113, and a sheet portion 116.

In the first aspect, in order to promote the filling of the solution 22 comprising a Japanese encephalitis vaccine and the solution 24 comprising at least one of an electrically neutral water-soluble polymer and a disaccharide into the needle recess portions 15, it is also preferable to carry out pressurization from the surface of the mold 13 and suction from the back surface of the mold 13 under reduced pressure.

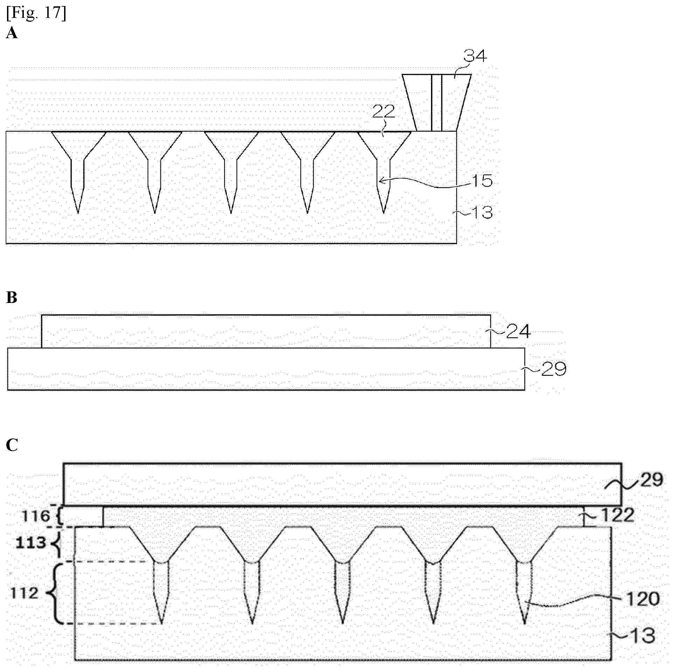

Next, a second aspect is described with reference to Fig. 17A to 17C. As shown in Fig. 17A, a solution 22 comprising a Japanese encephalitis vaccine is filled into the needle recess portions 15 of a mold 13 through a nozzle 34. Subsequently, as with Fig. 16B, the solution 22 comprising a Japanese encephalitis vaccine is dried and solidified, so that layers 120 comprising the Japanese encephalitis vaccine are formed in the needle recess portions 15. Subsequently, as shown in Fig. 17B, a solution 24 comprising at least one of an electrically neutral water-soluble polymer and a disaccharide is applied onto another support 29. The support 29 is not limited, and for example, polyethylene, polyethylene terephthalate, polycarbonate, polypropylene, acrylic resin, triacetyl cellulose, or glass can be used. Subsequently, as shown in Fig. 17C, the solution 24 formed on the support 29 is laminated on the mold 13 on which the layers 120 comprising the Japanese encephalitis vaccine are formed. Thereby, the solution 24 is filled into the needle recess portions 15. Since the layers comprising the Japanese encephalitis vaccine are solidified, diffusion of the Japanese encephalitis vaccine in the solution 24 can be suppressed. Subsequently, the solution 24 is dried and solidified, so as to form a microneedle array composed of a plurality of needle portions 112, frustum portions 113, and a sheet portion 116.

In the second aspect, in order to promote the filling of the solution 24 comprising at least one of an electrically neutral water-soluble polymer and a disaccharide into the needle recess portions 15, it is also preferable to carry out pressurization from the surface of the mold 13 and suction from the back surface of the mold 13 under reduced pressure.

The method of drying the solution 24 comprising at least one of an electrically neutral water-soluble polymer and a disaccharide may be a step of volatizing the solvent in the solution. The drying method is not particularly limited, and for example, methods such as heating, ventilation, or decompression can be used. The drying treatment can be carried out under conditions of 1°C to 50°C for 1 to 72 hours. In the case of ventilation, a method of blowing warm air to the solution at 0.1 to 10 m/sec is applied, for example. The drying temperature is preferably a temperature at which the Japanese encephalitis vaccine in the solution 22 comprising the Japanese encephalitis vaccine is not thermally deteriorated.

### (Peeling)

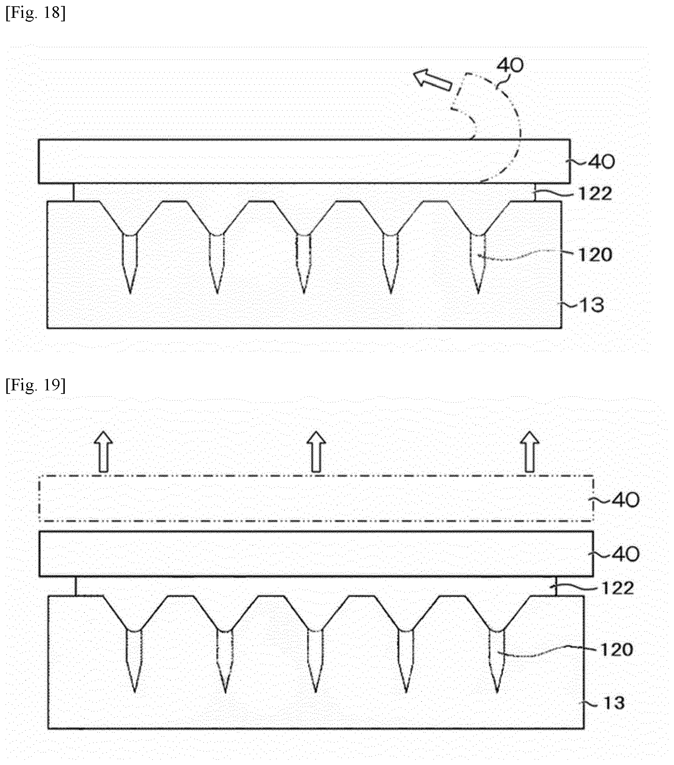

The method of peeling a microneedle array from a mold 13 is not particularly limited. Upon peeling, needle portions are preferably not bent or broken. Specifically, as shown in Fig. 18, a sheet-shaped base material 40 on which an adhesive layer having adhesiveness is formed is attached onto the microneedle array, and then, the base material 40 can be peeled from the edge. However, according to this method, the needle portions are likely to be bent. Thus, as shown in Fig. 19, a method comprising establishing a sucker (not shown in the figure) on the base material 40 on the microneedle array, and then pulling the base material up vertically, while sucking with air, can be applied. It is to be noted that a support 29 may be used as a base material 40.

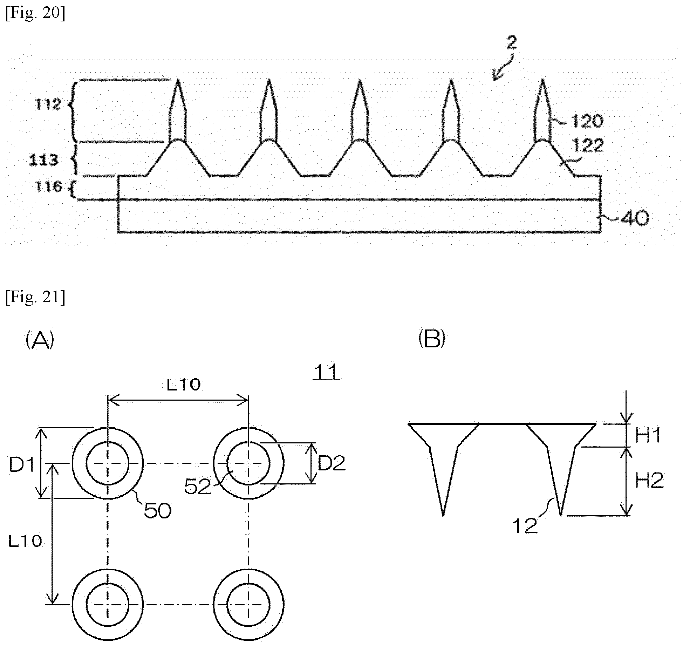

Fig. 20 shows a microneedle array 2 peeled from a mold 13. The microneedle array 2 is composed of a base material 40, and needle portions 112, frustum portions 113 and a sheet portion 116, which are formed on the base material 40. The needle portion 112 has a conical shape or a polygonal pyramidal shape, at least, at a tip thereof, but the shape of the needle portion 112 is not limited thereto.

The method for producing the microneedle array is not particularly limited. The microneedle array is preferably obtained by a production method comprising: (1) a step of producing a mold, (2) a step of preparing a solution comprising at least one of an electrically neutral water-soluble polymer and a disaccharide, a Japanese encephalitis vaccine, and an electrically neutral surfactant, (3) a step of filling the solution obtained in the step (2) into the mold to form needle portion tip regions, (4) a step of filling a solution comprising at least one of an electrically neutral water-soluble polymer and a disaccharide into the mold to form residual portions of the needle portions (frustum portions, as desired) and a sheet portion, and (5) a step of peeling a microneedle array from the mold.

The present invention will be more specifically described in the following examples. In the following examples, the material used, its amount and ratio, the details of the treatment, the treatment process, and the like may be suitably modified

Accordingly, the invention should not be restrictively interpreted by the specific examples mentioned below.

### Examples

Abbreviations and product names used in the following Examples have the following meanings.
HES: Hydroxyethyl Starch 70000 (Fresenius Kabi) (weight average molecular weight: 70000)
DEX: Dextran70000 (Meito Sangyo Co., Ltd.) (weight average molecular weight: 70000) CS: Sodium chondroitin sulfate (Maruha Nichiro Corporation) (weight average molecular weight: 90000)
Sucrose: Sucrose (FUJIFILM Wako Pure Chemical Corporation)
PVP K25: Polyvinylpyrrolidone (FUJIFILM Wako Pure Chemical Corporation) (weight average molecular weight: 24000)
Tw: MONTANOX (registered trademark) 80 API (Seppic)
SDS: Sodium dodecyl sulfate (FUJIFILM Wako Pure Chemical Corporation)
Pluronic (registered trademark) F-68 (NOF CORPORATION)
NIKKOL (registered trademark) HCO-60 (Nikko Chemicals Co., Ltd.)
Triton (registered trademark) X-100 (Alfa Aesar)
Sodium L-glutamate (FUJIFILM Wako Pure Chemical Corporation)

### [Example 1]

### < Production and evaluation of microneedle array >

### (Production of mold)

100 Needles, each consisting of a shaped portion 12 having a needle structure, in which a cone 52 having a diameter D2 of 300 µm and a height H2 of 500 µm was formed on a truncated cone 50 having a diameter D1 of 500 µm (bottom surface) and a height H1 of 150 µm, as shown in Fig. 21, were subjected to grinding processing to a square form with a pitch L1 of 1000 µm in a two-dimensional square array on the surface of a smooth Ni plate having a side of 40 mm, so as to produce an original form 11. On this original form 11, silicone rubber (SILASTIC MDX4-4210 manufactured by Dow Corning) was applied to form a film with a thickness of 0.6 mm, and 50 µm of a cone tip portion of the original form 11 that protruded from the film surface was then thermally cured, and was then peeled therefrom. Thereby, an inverted product of silicone rubber having a through hole with a diameter of approximately 30 µm was produced. This silicone rubber inverted product, in which two-dimensionally arrayed needle recess portions (10 columns x 10 lines) were formed in the center thereof and portions outside the planar section with a side of 30 mm were cut off, was used as a mold. The broader opening of a needle recess portion was set to be the surface of the mold, whereas the surface having a through hole (air vent hole) with a diameter of 30 µm was set to be the back surface of the mold.

### (Preparation of aqueous solution comprising Japanese encephalitis vaccine)

A stock solution of Japanese encephalitis vaccine (provided from BIKEN Group) was concentrated according to centrifugal ultrafiltration, and was then mixed with a water-soluble polymer, sucrose (FUJIFILM Wako Pure Chemical Corporation), sodium L-glutamate (FUJIFILM Wako Pure Chemical Corporation), and a surfactant. The amounts of individual components used are shown in Table 1.

### (Preparation of solution used to form sheet portion)

Hydroxyethyl starch 70000 (HES, Fresenius Kabi) was dissolved in water to prepare an aqueous solution of 56% by mass of HES.

### (Filling and drying of solution comprising Japanese encephalitis vaccine)

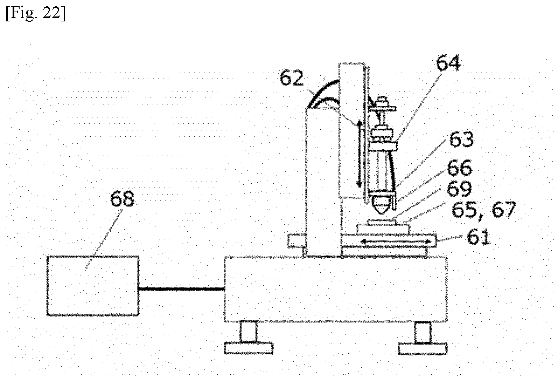

The filling device shown in Fig. 22 was used. The filling device comprises: an X-axis actuator 61 and a Z-axis actuator 62 that regulate relative position coordinates of a mold and a nozzle; a liquid supplying device 64 (manufactured by Musashi Engineering Inc., ultratrace determination dispenser SMP-III) in which a nozzle 63 can be equipped; a vacuum stand 65 on which a mold 69 is fixed; a laser displacement meter 66 for measuring the surface shape of the mold (manufactured by Panasonic Corporation, HL-C201A); a load cell 67 for measuring a nozzle pressing pressure (manufactured by Kyowa Electronic Instruments Co., Ltd., LCX-A-500N); and a control mechanism 68 that regulates the Z-axis based on the data of the measurement values of the surface shape and the pressing pressure.

A gas permeable film with a side of 15 mm (manufactured by Sumitomo Electric Industries, Ltd., Poreflon (registered trademark), FP-010) was placed on a horizontal vacuum stand, and a mold was placed thereon so that the surface thereof came to the top. From the direction of the back surface of the mold, pressure was reduced at a suction pressure (gauge pressure) of 90 kPa, so that the gas permeable film and the mold were fixed on the vacuum stand.

A nozzle made of SUS (stainless steel) having the shape shown in Fig. 13 was prepared, and a slit-shaped opening with a length of 12 mm and a width of 0.2 mm was formed on the center of a lip portion with a length of 20 mm and a width of 2 mm. This nozzle was connected with the liquid supplying device. 3 mL of Solution comprising a Japanese encephalitis vaccine was filled into the liquid supplying device and the nozzle. The nozzle was adjusted, so that the opening became parallel to a first line composed of a plurality of needle recess portions formed on the surface of the mold. The nozzle was pressed at a pressure of 1.372 × 10⁴ Pa (0.14 kgf/cm²) against the mold, at a position of 2 mm from the first line that was in the direction opposite to a second line. While the nozzle was pressed against the mold, the Z-axis was regulated so that a fluctuation in the pressing pressure was within ±0.490 × 10⁴ Pa (0.05 kgf/cm²). At the same time, while the liquid supplying device was moved at a rate of 0.5 m m/sec to the direction vertical to the length direction of the opening, the solution comprising a Japanese encephalitis vaccine was released from the opening of the liquid supplying device at a rate of 0.15 µL/sec for 20 seconds. The liquid supplying device passed through the pore patterns of the two-dimensionally arrayed multiple needle recess portions, and then, at a position 2 mm from the pore patterns, the moving of the nozzle was terminated, and the nozzle was removed from the mold.

The mold filled with the solution comprising a Japanese encephalitis vaccine was stored in a lid (box) in the environment of 23°C and 45%, and was then dried. At this time, the solution comprising a Japanese encephalitis vaccine was gradually dried, and after 180 minutes or longer had passed, the water content percentage became 20% or less. The drying means are not limited to the lid, but other means such as regulation of the temperature and the humidity or regulation of air volume may also be used.

### (Formation and drying of sheet portion)

The support used to form a sheet portion was prepared by subjecting a polyethylene terephthalate (PET) sheet (175 µm) to a hydrophilic plasma treatment using Cloud Remover (Victor jvc) under the following conditions (used gas: O₂; gas pressure: 13 Pa; high frequency (RF) electric power: 100 W; irradiation time: 3 minutes; O₂ flow rate: SV 250; and desired vacuum degree (CCG): 2.0 × 10⁻⁴ Pa). A solution used to form a sheet portion was applied onto the surface and back surface of the treated PET to result in each film thickness of 75 µm. On the other hand, the mold filled with the solution comprising a Japanese encephalitis vaccine was fixed on the vacuum stand by suction. The surface side of the PET sheet, onto which the solution used to form a sheet portion had been applied, was disposed facing with the surface of the mold, and further, the void between the PET sheet and the mold or the space existing on the side of the PET opposite to the mold was decompressed for 2 minutes. After completion of the decompression, only the space on the side of the PET opposite to the mold was exposed to the atmosphere, so that the PET sheet, onto which the solution used to form a sheet portion had been applied, was adhered to the mold. After the contacted state of the PET and the mold had been maintained for 10 minutes, an integrated body in which the PET adhered to the mold was dried under the environment of 23°C and 45% (relative humidity).

### (Peeling step)

The dried and solidified microneedle array was carefully peeled from the mold, so that a microneedle array comprising a Japanese encephalitis vaccine therein was formed. The present microneedle was composed of a frustum portion and a needle portion, and it was a truncated cone structure, in which the length L of a needle-shaped protrusion was approximately 800 µm, the width of a base portion was approximately 350 µm, the height of a frustum portion was approximately 190 µm, the diameter of the upper bottom surface was approximately 350 µm, and the diameter of the lower bottom surface was approximately 700 µm. The number of the microneedles was 109, and these microneedles were arranged with intervals of approximately 1 mm.

### [Examples 2 to 14 and Comparative Examples 1 and 2]

A microneedle array was produced in the same manner as that of Example 1, with the exception that the composition of an aqueous solution comprising a Japanese encephalitis vaccine and the composition of a solution used to form a sheet portion were changed as shown in Table 1.

The content of the Japanese encephalitis vaccine and the amount of the electrically neutral surfactant shown in Table 1 each indicate the amount thereof for a single microneedle array. The area of a single microneedle array was 1 cm². Besides, in Example 5, the microneedle array was produced at a ratio (mass ratio) of DEX/CS that was 70/30.

### (Evaluation 1: Vaccine activity after production of microneedle array)

A microneedle array as a whole was dissolved in 1 mL of a dissolving solution (a phosphate buffer supplemented with 0.1% of Tween (registered trademark) 20 and 0.5% of bovine serum albumin), and the vaccine activity was then evaluated according to ELISA (Enzyme-Linked ImmunoSorbent Assay). The expected content was calculated from (the concentration (µg/mL) of the Japanese encephalitis vaccine in the filling solution x the weight (mg) filled into the mold). The results of the above-described evaluation are shown in Table 1. The content of the vaccine detected by ELISA that was 80% or more of the expected content was evaluated as A, whereas the vaccine content that was less than 80% of the expected content was evaluated as B.

**[Table 1]**

| | Aqueous solution containing Japanese encephalitis vaccine | | | | | Solution for forming sheet portion | Vaccine activity after production |
|---|---|---|---|---|---|---|---|
| | Content of Japanese encephalitis vaccine | Water-soluble polymer | Disaccharides | Sodium L-glutamate | Surfactant | Water-soluble polymer | |
| Ex. 1 | 0.83 µg | - | Sucrose 750 µg | 208 µg | Tw 0.4 µg | HES | A |
| Comp. Ex. 1 | 0.83 µg | - | Sucrose 750 µg | 208 µg | - | HES | B |
| Ex. 2 | 0.83 µg | - | Sucrose 750 µg | 208 µg | Tw 2.2 µg | HES | A |
| Ex. 3 | 2.5 µg | - | Sucrose 750 µg | 208 µg | Tw 0.4 µg | HES | A |
| Ex. 4 | 2.5 µg | HES 100 µg | Sucrose 50 µg | 208 µg | Tw 2.2 µg | HES | A |
| Ex. 5 | 2.5 µg | HES 100 µg | Sucrose 50 µg | 208 µg | Tw 2.2 µg | DEX/CS | A |
| Ex. 6 | 0.83 µg | HES 100 µg | - | 208 µg | Tw 0.4 µg | HES | A |
| Ex. 7 | 0.83 µg | PVP K25 100 µg | Sucrose 750 µg | 208 µg | Tw 0.4 µg | HES | A |
| Comp. Ex. 2 | 0.83 µg | - | Sucrose 750 µg | 208 µ | SDS 2.2 µg | HES | B |
| Ex. 8 | 0.83 µg | - | Sucrose 750 µg | 208 µg | Pluronic F68 0.4 µg | HES | A |
| Ex. 9 | 0.83 µg | - | Sucrose 750 µg | 208 µg | Pluronic F68 2.2 µg | HES | A |
| Ex. 10 | 0.83 µg | - | Sucrose 750 µg | 208 µg | Pluronic F68 10 µg | HES | A |
| Ex. 11 | 0.83 µg | - | Sucrose 750 µg | 208 µg | HCO-60 0.4 µg | HES | A |
| Ex. 12 | 0.83 µg | - | Sucrose 750 µg | 208 µg | HCO-60 | HES | A |
| | | | | | 10 µg | | |
| Ex. 13 | 0.83 µg | - | Sucrose 750 µg | 208 µg | Triton-X 2.2 µg | HES | A |
| Ex. 14 | 0.25 µg | - | Sucrose 750 µg | 208 µg | Tw 2.2 µg | HES | A |

### Description of Reference Numerals

- 1: Microneedle array
- 2: Microneedle array
- 110: Microneedle
- 112: Needle portion
- 112A: Needle portion first layer
- 112B: Needle portion second layer
- 113: Frustum portion
- 116: Sheet portion
- 120: Layer containing Japanese encephalitis vaccine
- 122: Layer not containing Japanese encephalitis vaccine
- W: Diameter (width)
- H: Height
- T: Height (thickness)
- 11: Original form
- 12: Shaped portion
- 13: Mold
- 15: Needle recess portion
- 15A: Inlet
- 15B: Tip recess portion
- 15C: Air vent hole
- D: Diameter
- 18: Mold composite
- 19: Gas permeation sheet
- 20: Base
- 22: Japanese encephalitis vaccine-containing solution
- 24: Solution containing at least one of electrically neutral water-soluble polymer and disaccharide
- 29: Support
- 30: Tank
- 32: Pipe
- 34: Nozzle
- 34A: Lip portion
- 34B: Opening
- 36: Liquid supplying device
- P1: Pressurization power
- P2: Pressing force
- P3: Pressing force
- 40: Base material
- 50: Truncated cone
- 52: Cone
- D1: Diameter
- D2: Diameter
- L10: Pitch
- H1: Height
- H2: Height
- 61: X-axis actuator
- 62: Z-axis actuator
- 63: Nozzle
- 64: Liquid supplying device
- 65: Vacuum stand
- 66: Laser displacement meter
- 67: Load cell
- 68: Control mechanism
- 69: Mold

## Claims

1. A microneedle array (1) having a sheet portion (116) and a plurality of needle portions (112) present on the upper surface of the sheet portion (116), wherein
the needle portion (112) comprises at least one of an electrically neutral water-soluble polymer and a disaccharide, and wherein the needle portion (112) further comprises a Japanese encephalitis vaccine and an electrically neutral surfactant, and
the sheet portion (116) comprises at least one of an electrically neutral water-soluble polymer and a disaccharide.

2. The microneedle array (1) according to claim 1, wherein the electrically neutral surfactant is sorbitan fatty acid ester, polyoxyethylene sorbitan fatty acid ester, a polyoxyethylene/polyoxypropylene copolymer, polyoxyethylene hydrogenated castor oil, or octylphenol ethoxylate.

3. The microneedle array (1) according to claim 1 or 2, wherein the weight average molecular weight of the electrically neutral water-soluble polymer comprised in the needle portion (112) is 5,000 or more and 100,000 or less.

4. The microneedle array (1) according to any one of claims 1 to 3, wherein the water-soluble polymer comprised in the needle portion (112) is a polysaccharide.

5. The microneedle array (1) according to any one of claims 1 to 4, wherein the water-soluble polymer comprised in the needle portion (112) is hydroxyethyl starch.

6. The microneedle array (1) according to any one of claims 1 to 5, wherein the weight average molecular weight of the electrically neutral water-soluble polymer comprised in the sheet portion (116) is 5,000 or more and 100,000 or less.

7. The microneedle array (1) according to any one of claims 1 to 6, wherein the water-soluble polymer comprised in the sheet portion (116) is a polysaccharide.

8. The microneedle array (1) according to any one of claims 1 to 7, wherein the water-soluble polymer comprised in the sheet portion (116) is hydroxyethyl starch.

9. The microneedle array (1) according to any one of claims 1 to 8, wherein the disaccharide comprised in the needle portion (112) is sucrose.

## Patentansprüche

1. Mikronadelanordnung (1) mit einem Blattteil (116) und einer Vielzahl von Nadelteilen (112), die auf einer oberen Oberfläche des Blattteils (116) vorliegen, worin
der Nadelteil (112) zumindest eines von einem elektrisch neutralen, wasserlöslichen Polymer und einem Disaccharid umfasst, und worin der Nadelteil (112) ferner einen Impfstoff gegen japanische Encephalitis und ein elektrisch neutrales Tensid umfasst, und
worin der Blattteil (116) zumindest eines von einem elektrisch neutralen, wasserlöslichen Polymer und einem Disaccharid umfasst.

2. Mikronadelanordnung (1) gemäß Anspruch 1, worin das elektrisch neutrale Tensid Sorbitanfettsäureester, Polyoxyethylensorbitanfettsäureester, Polyoxyethylen/Polyoxypropylen-Copolymer, Polyoxyethylen-hydriertes Rizinusöl oder Octylphenolethoxylat ist.

3. Mikronadelanordnung (1) gemäß Anspruch 1 oder 2, worin das gewichtsgemittelte Molekulargewicht des elektrisch neutralen, wasserlöslichen Polymers, das im Nadelteil (112) umfasst ist, 5.000 oder größer und 100.000 oder kleiner ist.

4. Mikronadelanordnung (1) gemäß irgendeinem der Ansprüche 1 bis 3, worin das in dem Nadelteil (112) umfasste wasserlösliche Polymer ein Polysaccharid ist.

5. Mikronadelanordnung (1) gemäß irgendeinem der Ansprüche 1 bis 4, worin das in dem Nadelteil (112) umfasste wasserlösliche Polymer Hydroxyethyl-Stärke ist.

6. Mikronadelanordnung (1) gemäß irgendeinem der Ansprüche 1 bis 5, worin das gewichtsgemittelte Molekulargewicht des in dem Blattteil (116) umfassten elektrisch neutralen, wasserlöslichen Polymers 5.000 oder größer und 100.000 oder kleiner ist.

7. Mikronadelanordnung (1) gemäß irgendeinem der Ansprüche 1 bis 6, worin das in dem Blattteil (116) umfasste wasserlösliche Polymer ein Polysaccharid ist.

8. Mikronadelanordnung (1) gemäß irgendeinem der Ansprüche 1 bis 7, worin das in dem Blattteil (116) umfasste wasserlösliche Polymer Hydroxyethyl-Stärke ist.

9. Mikronadelanordnung (1) gemäß irgendeinem der Ansprüche 1 bis 8, worin das im Nadelteil (112) umfasste Disaccharid Saccharose ist.

## Revendications

1. Réseau (1) de micro-aiguilles disposant d'une partie feuille (116) et d'une pluralité de parties aiguilles (112) présentes sur la surface supérieure de la partie feuille (116), dans lequel
la partie aiguille (112) comprend au moins l'un parmi un polymère hydrosoluble neutre électriquement et un disaccharide, et dans lequel la partie aiguille (112) comprend en outre un vaccin contre l'encéphalite japonaise et un tensioactif neutre électriquement, et
la partie feuille (116) comprend au moins l'un parmi un polymère hydrosoluble neutre électriquement et un disaccharide.

2. Réseau (1) de micro-aiguilles selon la revendication 1, dans lequel le tensioactif neutre électriquement est un ester d'acide gras de sorbitane, un ester d'acide gras de sorbitane polyoxyéthylène, un copolymère de polyoxyéthylène/polyoxypropylène, une huile de ricin hydrogénée de polyoxyéthylène ou un éthoxylate d'octylphénol.

3. Réseau (1) de micro-aiguilles selon la revendication 1 ou la revendication 2, dans lequel le poids moléculaire moyen en poids du polymère hydrosoluble neutre électriquement compris dans la partie aiguille (112) est de 5 000 ou plus et de 100 000 ou moins.

4. Réseau (1) de micro-aiguilles selon l'une quelconque des revendications 1 à 3, dans lequel le polymère hydrosoluble compris dans la partie aiguille (112) est un polysaccharide.

5. Réseau (1) de micro-aiguilles selon l'une quelconque des revendications 1 à 4, dans lequel le polymère hydrosoluble compris dans la partie aiguille (112) est de l'amidon hydroxyéthylé.

6. Réseau (1) de micro-aiguilles selon l'une quelconque des revendications 1 à 5, dans lequel le poids moléculaire moyen en poids du polymère hydrosoluble neutre électriquement compris dans la partie feuille (116) est de 5 000 ou plus et de 100 000 ou moins.

7. Réseau (1) de micro-aiguilles selon l'une quelconque des revendications 1 à 6, dans lequel le polymère hydrosoluble compris dans la partie feuille (116) est un polysaccharide.

8. Réseau (1) de micro-aiguilles selon l'une quelconque des revendications 1 à 7, dans lequel le polymère hydrosoluble compris dans la partie feuille (116) est de l'amidon hydroxyéthylé.

9. Réseau (1) de micro-aiguilles selon l'une quelconque des revendications 1 à 8, dans lequel le disaccharide compris dans la partie aiguille (112) est du saccharose.
